# EUROPEAN PATENT SPECIFICATION

(11) **EP 1 987 001 B1**
(45) Date of publication and mention of the grant of the patent: **05.05.2010**
(21) Application number: 07726356.4
(22) Date of filing: 13.02.2007
(51) Int. Cl.: C07D 211/32, C07D 407/12, A61K 31/4525, A61K 31/445, A61P 25/00

(54) **1-PHENETHYLPIPERIDINE DERIVATIVES AND THEIR USE AS OPIOID RECEPTOR LIGANDS**
1-PHENETHYLPIPERIDINDERIVATE UND IHRE VERWENDUNG ALS OPIOIDREZEPTOR-LIGANDEN
DÉRIVÉS DE 1-PHÉNÉTHYLPIPÉRIDINE ET LEURS UTILISATIONS EN TANT QUE LIGANDS DE RÉCEPTEUR OPIOÏDE DE TYPE

(30) Priority: 17.02.2006 DK 200600232; 21.02.2006 US 774668 P
(43) Date of publication of application: 05.11.2008
(73) Proprietor: NeuroSearch A/S, 2750 Ballerup (DK)
(72) Inventor: PETERS, Dan, DK-2750 Ballerup (DK); ERIKSEN, Birgitte, L., DK-2750 Ballerup (DK); MUNRO, Gordon, DK-2750 Ballerup (DK); NIELSEN, Elsebet, Østergaard, DK-2750 Ballerup (DK); REDROBE, John, Paul, DK-2750 Ballerup (DK)
(74) Representative: Abildgren, Michael Padkjaer
(86) International application number: PCT/EP2007/051400
(87) International publication number: WO 2007/093603

(56) References cited:
- EP-A1- 0 457 686
- WO-A-01/81308
- WO-A-2004/035541

## Description

### TECHNICAL FIELD

This invention relates to novel 1-phenethylpiperidine derivatives useful as opioid receptor ligands. More specifically, the invention provides compounds useful as µ opioid receptor ligands.

In other aspects the invention relates to these compounds for use in a method for therapy, such as for the treatment of pain, and to pharmaceutical compositions comprising the compounds of the invention.

### BACKGROUND ART

Numerous classes of opioid receptors exist. These classes differ in their affinity for various opioid ligands and in their cellular and organ distribution. Moreover, although the different classes are believed to serve different physiological functions, there is a substantial overlap of function, as well as distribution. Three different types of opioid receptors have been identified, the mu (µ), delta (δ) and kappa (κ) opioid receptor. These three opioid receptor types are the sites of action of opioid ligands producing analgesic effects. However, the type of pain inhibited and the secondary functions vary with each receptor type. The µ receptor is generally regarded as primarily associated with pain relief, and drug or other chemical dependence, such as addiction or alcoholism. The δ receptor appears to deal with behavioural effects, although the δ and the κ receptors may also mediate analgesia.

Each opioid receptor, when coupled with an opiate, causes a specific biological response unique to that type of receptor. When an opiate activates more than one receptor, the biological response for each receptor is affected, thereby producing side effects. The less specific and selective an opiate may be, the greater the chance of causing increased side effect by the administration of the opiate.

Whereas morphine, which is a strong opioid analgetic agent shows effectiveness against strong pain by acting on the µ opioid receptor (agonist activity), there is a problem that its side effects such as nausea and neurologic manifestation including hallucination and derangement. Moreover, morphine forms psychological dependence, causing serious problems. Other side effects reported are respiratory depression, tolerance, physical dependence capacity, and precipitated withdrawal syndrome, caused by non-specific interactions with central nervous receptors.

WO 03/004026 describes substituted 1-phenethylpiperidine compounds used as inter alia analgesics.

### SUMMARY OF THE INVENTION

It is an object of the invention to provide novel compounds which act on opiate receptors.

A further object of the invention is the provision of compounds that substantially avoid the unwanted side effects associated with conventional peripherally acting analgesics.

It is a further object to provide compounds that bind selectively to the µ opioid receptor.

A still further object is the provision of compounds that in addition show activity as monoamine neurotransmitter re-uptake inhibitors.

In its first aspect, the invention provides a compound of Formula I, 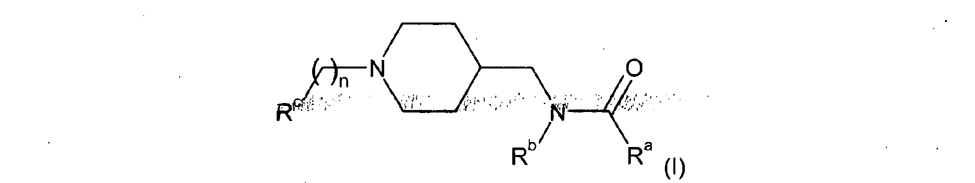 any of its isomers or any mixture of its isomers, or a pharmaceutically acceptable salt thereof; wherein n, R^{a}, R^{b} and R^{c} are as defined below.

In its second aspect, the invention provides a pharmaceutical composition, comprising a therapeutically effective amount of a compound of the invention, any of its isomers or any mixture of its isomers, or a pharmaceutically acceptable salt thereof, together with at least one pharmaceutically acceptable carrier, excipient or diluent.

In a further aspect, the invention provides the compound of the invention, any of its isomers or any mixture of its isomers, or a pharmaceutically acceptable salt thereof, for the manufacture of a pharmaceutical composition for the treatment, prevention or alleviation of a disease or a disorder or a condition of a mammal, including a human, which disease, disorder or condition is responsive to modulation of the opioid receptor.

In a still further aspect, the invention relates to a compound of formula (I) for use in a method for treatment, prevention or alleviation of a disease or a disorder or a condition of a living animal body, including a human, which disorder, disease or condition is responsive to responsive to modulation of the opioid receptor, which method comprises the step of administering to such a living animal body in need thereof a therapeutically effective amount of a compound of the invention, any of its isomers or any mixture of its isomers, or a pharmaceutically acceptable salt thereof.

### DETAILED DISCLOSURE OF THE INVENTION

### 1-phenethylpiperidine derivatives

In its first aspect, the invention provides a compound of Formula I, 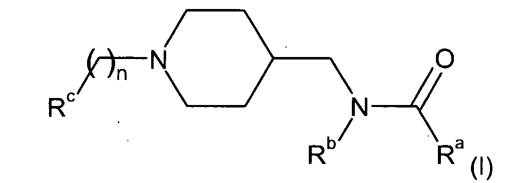 any of its isomers or any mixture of its isomers, or a pharmaceutically acceptable salt thereof;
wherein
n is 1 or 2;
R^{a} represents hydrogen, alkyl or R^{d};
   which alkyl is optionally substituted with one or more substituents independently selected from the group consisting of:
   halo, trifluoromethyl, trifluoromethoxy, cyano, hydroxy, amino, nitro, alkoxy, cycloalkoxy, alkyl, cycloalkyl, cycloalkylalkyl, alkenyl and alkynyl;
R^{b} represents R^{e} or -CH₂-R^{e};
R^{c} represents R^{f}, -CH=CH-R^{f} or -CH=CH₂; and
R^{d}, R^{e} and R^{f} independent of each other represent an aryl group;
   which aryl group is optionally substituted with one or more substituents independently selected from the group consisting of:
   halo, trifluoromethyl, trifluoromethoxy, cyano, nitro, hydroxy, alkoxy, cycloalkoxy, alkoxyalkyl, cycloalkoxyalkyl, methylenedioxy, ethylenedioxy, alkyl, cycloalkyl, cycloalkylalkyl, alkenyl, alkynyl, sulfanyl, thioalkoxy, -NR'R", -(C=O)NR'R" or -NR'(C=O)R";
wherein R' and R" independent of each other are hydrogen or alkyl.

In a further aspect, the invention provides a compound of Formula II, 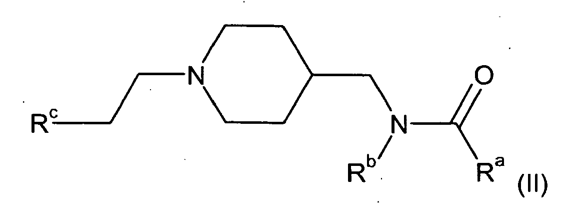 any of its isomers or any mixture of its isomers, or a pharmaceutically acceptable salt thereof, wherein
R^{a} represents hydrogen, alkyl or R^{d};
   which alkyl is optionally substituted with one or more substituents independently selected from the group consisting of:
   halo, trifluoromethyl, trifluoromethoxy, cyano, hydroxy, amino, nitro, alkoxy, cycloalkoxy, alkyl, cycloalkyl, cycloalkylalkyl, alkenyl and alkynyl; and
R^{b}, R^{c} and R^{d} independent of each other represent an aryl group;
   which aryl group is optionally substituted with one or more substituents independently selected from the group consisting of:
   halo, trifluoromethyl, trifluoromethoxy, cyano, nitro, hydroxy, alkoxy, cycloalkoxy, alkoxyalkyl, cycloalkoxyalkyl, methylenedioxy, ethylenedioxy, alkyl, cycloalkyl, cycloalkylalkyl, alkenyl, alkynyl, sulfanyl, thioalkoxy, -NR'R", -(C=O)NR'R" or -NR'(C=O)R";
wherein R' and R" independent of each other are hydrogen or alkyl.

In one embodiment of the compound of formula I, n is 1. In a second embodiment, n is 2.

In a further embodiment of the compound of formula I, R^{a} represents alkyl. In a special embodiment, R^{a} represents methyl or ethyl.

In a still further embodiment of the compound of formula I, R^{b} represents an optionally substituted aryl group, such as an optionally substituted phenyl. In a special embodiment, R^{b} represents phenyl. In a further embodiment, R^{b} represents halophenyl, such as fluorophenyl, chlorophenyl, 4-halophenyl or 3-halophenyl, in particular 4-chlorophenyl, 4-fluorophenyl or 3-fluorophenyl. In a further embodiment, R^{b} represents alkoxyphenyl, such as 4-alkoxyphenyl, 3-alkoxyphenyl or methoxyphenyl, in particular 4-methoxyphenyl or 3-methoxyphenyl. In a still further embodiment, R^{b} represents phenyl substituted with methylenedioxy, such as benzo[1,3]dioxol-5-yl.

In a further embodiment of the compound of formula I, R^{b} represents -CH₂-R^{e}. In a special embodiment, R^{b} represents benzyl.

In a still further embodiment of the compound of formula I, R^{c} represents an optionally substituted aryl group. In a special embodiment, R^{c} represents phenyl. In a further embodiment, R^{c} represents halophenyl, such as fluorophenyl, chlorophenyl, 4-halophenyl or 3-halophenyl, in particular 4-chlorophenyl, 4-fluorophenyl or 3-fluorophenyl. In a still further embodiment, R^{c} represents alkoxyphenyl, such as 4-alkoxyphenyl or methoxyphenyl, in particular 4-methoxyphenyl.

In a further embodiment of the compound of formula I, R^{c} represents -CH=CH-R^{f}. In a special embodiment, R^{f} represents phenyl.

In a still further embodiment of the compound of formula I, R^{c} represents -CH=CH₂.

In a special embodiment the compound of the invention is
*N*-(4-Chloro-phenyl)-*N*-(1-phenethyl-piperidin-4-ylmethyl)-propionamide;
*N*-(3-Methoxy-phenyl)-*N*-(1-phenethyl-piperidin-4-ylmethyl)-propionamide;
*N*-Benzo[1,3]dioxol-5-yl-*N*-(1-phenethyl-piperidin-4-ylmethyl)-propionamide;
*N*-Benzo[1,3]dioxol-5-yl-*N*-{1-[2-(4-chloro-phenyl)-ethyl]-piperidin-4-ylmethyl}-propionamide;
*N*-(4-Methoxy-phenyl)-*N*-(1-phenethyl-piperidin-4-ylmethyl)-propionamide;
*N*-{1-[2-(4-Chloro-phenyl)-ethyl]-piperidin-4-ylmethyl}-*N*-(4-methoxy-phenyl)-propionamide;
*N*-{1-[2-(4-Chloro-phenyl)-ethyl]-piperidin-4-ylmethyl}-*N*-(3-methoxy-phenyl)-propionamide ;
*N*-(1-But-3-enyl-piperidin-4-ylmethyl)-N-phenyl-propionamide;
*N*-Phenyl-*N*-[1-((E)-3-phenyl-allyl)-piperidin-4-ylmethyl]-propionamide;
*N*-{1-[2-(4-Fluoro-phenyl)-ethyl]-piperidin-4-ylmethyl}-*N*-phenyl-propionamide;
*N*-{1-[2-(-Fluoro-phenyl)-ethyl]-piperidin-4-ylmethyl}-*N*-phenyl-propionamide;
*N*-(4-Fluoro-phenyl)-*N*-(1-phenethyl-piperidin-4-ylmethyl)-propionamide;
*N*-(4-Fluoro-phenyl)-*N*-{1-[2-(3-fluoro-phenyl)-ethyl]-piperidin-4-ylmethyl}-propionamide;
*N*-(4-Fluoro-phenyl)-*N*-{1-[2-(4-methoxy-phenyl)-ethyl]-piperidin-4-ylmethyl}-propionamide;
*N*-(4-Fluoro-phenyl)-*N*-{1-[2-(4-fluoro-phenyl)-ethyl]-piperidin-4-ylmethyl}-propionamide;
*N*-(3-Fluoro-phenyl)-*N*-(1-phenethyl-piperidin-4-ylmethyl)-propionamide;
*N*-(3-Fluoro-phenyl)-*N*-{1-[2-(4-fluoro-phenyl)-ethyl]-piperidin-4-ylmethyl}-propionamide;
*N*-(3-Fluoro-phenyl)-*N*-{1-[2-(3-fluoro-phenyl)-ethyl]-piperidin-4-ylmethyl}-propionamide;
*N*-Benzyl-*N*-(1-phenethyl-piperidin-4-ylmethyl)-propionamide;
*N*-(1-Phenethyl-piperidin-4-ylmethyl)-*N*-phenyl-acetamide;
*N*-(1-Phenethyl-piperidin-4-yl-methyl)-*N*-phenyl-propionamide;
*N*-(1-Benzyl-piperidin-4-ylmethyl)-*N*-phenyl-propionamide;
*N*-[1-(4-Chloro-benzyl)-piperidin-4-ylmethyl]-*N*-phenyl-propionamide;
*N*-{1-[2-(4-Chloro-phenyl)-ethyl]-piperidin-4-ylmethyl}-*N*-phenyl-propionamide;
or a pharmaceutically acceptable salt thereof.

Any combination of two or more of the embodiments described herein is considered within the scope of the present invention.

### Definition of Substituents

In the context of this invention halo represents fluoro, chloro, bromo or iodo.

In the context of this invention an alkyl group designates a univalent saturated, straight or branched hydrocarbon chain. The hydrocarbon chain preferably contains of from one to six carbon atoms (C₁₋₆-alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

In the context of this invention an alkenyl group designates a carbon chain containing one or more double bonds, including di-enes, tri-enes and poly-enes. In a preferred embodiment the alkenyl group of the invention comprises of from two to six carbon atoms (C₂₋₆-alkenyl), including at least one double bond. In a most preferred embodiment the alkenyl group of the invention is ethenyl; 1- or 2-propenyl; 1-, 2- or 3-butenyl, or 1,3-butadienyl; 1-, 2-, 3-, 4- or 5-hexenyl, or 1,3-hexadienyl, or 1,3,5-hexatrienyl.

In the context of this invention an alkynyl group designates a carbon chain containing one or more triple bonds, including di-ynes, tri-ynes and poly-ynes. In a preferred embodiment the alkynyl group of the invention comprises of from two to six carbon atoms (C₂₋₆-alkynyl), including at least one triple bond. In its most preferred embodiment the alkynyl group of the invention is ethynyl; 1-, or 2-propynyl; 1-, 2-, or 3- butynyl, or 1,3-butadiynyl; 1-, 2-, 3-, 4-pentynyl, or 1,3-pentadiynyl; 1-, 2-, 3-, 4-, or 5- hexynyl, or 1,3-hexadiynyl or 1,3,5-hexatriynyl.

In the context of this invention a cycloalkyl group designates a cyclic alkyl group, preferably containing of from three to seven carbon atoms (C₃₋₇-cycloalkyl), including cyclopropyl, cyclobutyl, cyclopentyl, cyclohexyl and cycloheptyl.

Alkoxy is -O-alkyl, wherein alkyl is as defined above.

Cycloalkoxy means -O-cycloalkyl, wherein cycloalkyl is as defined above.

Cycloalkylalkyl means cycloalkyl as above and alkyl as above, meaning for example, cyclopropylmethyl.

Thioalkoxy is -S-alkyl, wherein alkyl is as defined above.

In the context of this invention an aryl group designates a carbocyclic aromatic ring system such as phenyl, naphthyl (1-naphthyl or 2-naphthyl) or fluorenyl.

### Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysinium, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

The chemical compound of the invention may be provided in dissoluble or indissoluble forms together with a pharmaceutically acceptable solvent such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

### Steric Isomers

It will be appreciated by those skilled in the art that the compounds of the present invention may exist in different stereoisomeric forms - including enantiomers, diastereomers and cis-trans-isomers.

The invention includes all such isomers and any mixtures thereof including racemic mixtures.

Methods for the resolvation of optical isomers, known to those skilled in the art may be used, and will be apparent to the average worker skilled in the art. Such methods include those discussed by J. Jaques, A. Collet, and S. Wilen in "Enantiomers, Racemates, and Resolutions", John Wiley and Sons, New York (1981).

Optical active compounds can also be prepared from optical active starting materials.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹³C, ¹⁴C, ¹³¹I, ¹²⁵I, ¹²³I, and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

### Methods of Preparation

The chemical compounds of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

Compounds of the invention may be tested for their ability to bind to the µ, δ, and κ opioid receptors, e.g. such as described by Simonin F et al [Simonin F et al, Mol. Pharmacol., 46(6), 1015-21, 1994], Simonin F et al [Simonin F et al, Proc. Natl. Acad. Sci. USA, 92(15), 7006-10, 1995], and Wang JB et al [Wang JB et al, FEBS Lett., 348(1), 75-9, 1994].

Compounds that bind to opiate receptors, in particular the µ receptor, are likely to be useful in the treatment of pain, postoperative pain, chronic pain (such as cancer pain and neuropathic pain), pain during labour and delivery, migraine, drug addiction (such as heroin addiction and cocaine addiction), and alcoholism.

Furthermore, compounds that bind to oplate receptors are also likely to be useful in the treatment of irritable bowel syndrome, constipation, nausea, vomiting, and pruritic dermatoses (itching), such as allergic dermatitis and atopy. Compounds that bind to opiate receptors have also been indicated in the treatment of eating disorders, opiate overdoses, depression, smoking, sexual dysfunction, shock, stroke, spinal damage, head trauma, diarrhoea, urinary incontinence and inflammatory reactions.

Thus in further aspect, the compounds of the invention are considered useful for the treatment, prevention or alleviation of a disease, disorder or condition responsive to modulation of the opioid receptors, in particular the µ opioid receptor.

In a special embodiment, the compounds of the invention are considered useful for the treatment, prevention or alleviation of pain, postoperative pain, chronic pain, cancer pain, neuropathic pain, pain during labour and delivery, migraine, drug addiction, heroin addiction, cocaine addiction, alcoholism, irritable bowel syndrome, constipation, nausea, vomiting, pruritic dermatoses, allergic dermatitis, atopy, eating disorders, opiate overdoses, depression, smoking, sexual dysfunction, shock, stroke, spinal damage, head trauma, diarrhoea, urinary incontinence and inflammatory reactions.

In a further embodiment, the compounds of the invention are considered particularly useful for the treatment, prevention or alleviation of pain, postoperative pain, chronic pain, migraine, drug addiction, alcoholism, and irritable bowel syndrome.

In a still further embodiment, the compounds of the invention also show activity as monoamine neurotransmitter re-uptake inhibitors. The compounds of the invention may be tested for their ability to inhibit reuptake of the monoamines dopamine, noradrenaline and serotonin in synaptosomes e.g. such as described in WO 97/30997.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

### Pharmaceutical Compositions

In another aspect the invention provides novel pharmaceutical compositions comprising a therapeutically effective amount of a compound of the invention.

While a compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising a compound of the invention, or a pharmaceutically acceptable salt, together with one or more pharmaceutically acceptable carriers, and, optionally, other therapeutic and/or prophylactic ingredients, known and used in the art. The carrier(s) must be "acceptable" in the sense of being compatible with the other ingredients of the formulation and not harmful to the recipient thereof.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient, which ameliorates the symptoms or condition. Therapeutic efficacy and toxicity, e.g. ED₅₀ and LD₅₀, may be determined by standard pharmacological procedures in cell cultures or experimental animals. The dose ratio between therapeutic and toxic effects is the therapeutic index and may be expressed by the ratio LD₅₀/ED₅₀. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 i.v. and 100 mg/kg p.o. Preferred ranges are from about 0.1 µg/kg to about 10 mg/kg/day i.v., and from about 1 µg/kg to about 100 mg/kg/day p.o.

### Methods of Therapy

In another aspect the invention provides a compound of formula (I) for use in a method for the treatment, prevention or alleviation of a disease or a disorder or a condition of a living animal body, including a human, which disease, disorder or condition is responsive to modulation of the the opioid receptor, and which method comprises administering to such a living animal body, including a human, in need thereof an effective amount of a compound of the invention, any of its isomers or any mixture of its isomers, or a pharmaceutically acceptable salt thereof.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

### EXAMPLES

The invention is further illustrated with reference to the following examples, which are not intended to be in any way limiting to the scope of the invention as claimed.

### Example 1

### Piperidine-1,4-dicarboxylic acid 1-benzyl ester 4-ethyl ester (1)

Ethyl 4-piperidinecarboxylate (99.48 g, 632,78 mmol) and pyridine (56.8 g, 696 mmol) were dissolved in dichloromethane (800 mL) and cooled to 0°C. Benzyl chloroformate (88.1 mL, 601 mmol) was added dropwise and the reaction mixture was allowed to reach room temperature overnight. The reaction was quenched with aqueous hydrochloric acid (1 M), extracted with dichloromethane, washed with brine, dried over sodium sulphate, filtered and concentrated to give piperidine-1,4-dicarboxylic acid 1-benzyl ester 4-ethyl ester (165 g, 89%) as a yellow oil.

### Example 2

### 4-Hydroxymethyl-piperidine-1-carboxylic acid benzyl ester (2)

Piperidine-1,4-dicarboxylic acid 1-benzyl ester 4-ethyl ester (1) (165 g, 567. mmol) was dissolved in tetrahydrofuran (1500 mL) and cooled to 0°C. Lithium aluminium hydride (311 mL, 1 M in THF) was added dropwise maintaining the temperature below 5°C. The reaction mixture was stirred for one hour and aqueous sodium hydroxide (4 N) was added dropwise. The mixture was filtered and the filtercake was washed with diethyl ether. The organic layer was concentrated and dissolved in ethyl acetate, washed with water and brine, dried over sodium sulphate, filtered and concentrated to give 4-hydroxymethyl-piperidine-1-carboxylic acid benzyl ester (120 g, 85%) as a yellow oil.

### Example 3

### 4-Formyl-piperidine-1-carboxylic acid benzyl ester (3)

Oxalyl chloride (59.1 mL, 674 mmol) was dissolved in dichloromethane (500 mL) and cooled to -78°C. Dimethylsulfoxide (68.3 mL, 963 mmol) was added and the mixture was stirred for 15 min. 4-Hydroxymethyl-piperidine-1-carboxylic acid benzyl ester (2) (120 g, 481 mmol) dissolved in dichloromethane (500 mL) was added. The mixture was allowed to warm to -55°C for 15 min. The mixture was again cooled to -78°C and triethylamine (205 mL, 1443 mmol) in dichloromethane (250 mL) was added. The suspension was allowed to warm to room temperature and quenched with glacial acetic acid (100 mL). The solution was washed with water and the aqueous phase extracted with dichloromethane (2 x 200 mL). The combined organic layers were washed with brine, dried over sodium sulphate, filtered and concentrated to give 4-formyl-piperidine-1-carboxylic acid benzyl ester (119 g, 100%).

### Example 4

### 4-[(3-Fluoro-phenylamino)-methyl]-piperidine-1-carboxylic acid benzyl ester (4)

4-Formyl-piperidine-1-carboxylic acid benzyl ester (3) (2.00 g, 8.09 mmol) and 3-fluoroaniline (1.16 mL, 12.1 mmol) was dissolved in 1,2-dichloroethane (40 mL). Sodium sulphate (5.74 g, 40.4 mmol) and sodium triacetoxyborohydride (2.74 g, 12.9 mmol) were added and the reaction was stirred at room temperature overnight. Aqueous sodium hydroxide was added carefully and the reaction mixture was stirred for an additional half hour followed by extraction with dichloromethane. The organic phases were washed with brine, dried over sodium sulphate, filtered and concentrated. The crude product was purified by flash chromatography using ethyl acetate/heptane as eluent to give 4-[(3-fluoro-phenylamino)-methyl]-piperidine-1-carboxylic acid benzyl ester (380 mg, 14%)

### Example 5

### 4-{[(3-Fluoro-phenyl)-propionyl-amino]-methyl}-piperidine-1-carboxylic acid benzyl ester (5)

4-[(3-Fluoro-phenylamino)-methyl]-piperidine-1-carboxylic acid benzyl ester (4) (1.12 g, 3.27 mmol) and triethyl amine (0.70 mL, 4.90 mmol) were dissolved in dichloromethane (50 mL) and the mixture was cooled to 0°C. Propionyl chloride (0.38 mL, 4.25 mmol) was added and the reaction was stirred at room temperature overnight. Aqueous sodium hydrogen carbonate was added and the aqueous phase was extracted with dichloromethane. The combined organic phases were washed with brine, dried over sodium sulphate, filtered and concentrated. The crude product was purified by flash chromatography using ethyl acetate/heptane as eluent to give 4-{[(3-fluoro-phenyl)-propionyl-amino]-methyl}-piperidine-1-carboxylic acid benzyl ester (800 mg, 61 %).

### Example 6

### N-(3-Fluoro-phenyl)-N-piperidin-4-ylmethyl-propionamide (6)

4-{[(3-Fluoro-phenyl)-propionyl-amino]-methyl}-piperidine-1-carboxylic acid benzyl ester (5) (800 mg, 2.01 mmol) was dissolved in ethanol (30 mL) and palladium (10% on activated carbon) was added. The reaction mixture was stirred under a hydrogen atmosphere for 3 hours. The mixture was filtered through a pad of kieselguhr and the solution concentrated *in vacuo* to give N-(3-fluoro-phenyl)-N-piperidin-4-ylmethylpropionamide (480 mg, 90%) as a colorless oil.

### Example 7

### 4-[(Benzyl-propionyl-amino)-methyl]-piperidine-1-carboxylic acid tert-butyl ester (7)

4-(Propionylamino-methyl)-piperidine-1-carboxylic acid *tert*-butyl ester (9b) (530 mg, 1.96 mmol) was dissolved in tetrahydrofuran (10 mL) and cooled to 0°C. Sodium hydride (86 mg, 2.16 mmol) was added portion wise and the reaction mixture was stirred for 45 min at 0°C. Benzyl chloride (0.24 mL, 2.35 mmol) was added and the reaction mixture was heated to reflux over night. Aqueous sodium hydrogen carbonate was added and the aqueous phase was extracted with ethyl acetate. The combined organic phases were washed with brine, dried over sodium sulphate filtered and concentrated *in vacuo* to give 4-[(benzyl-propionyl-amino)-methyl]-piperidine-1-carboxylic acid *tert*-butyl ester (900 mg, 100%) as a yellow oil.

### Example 8

### 4-[(4-Chloro-phenylamino)-methyl]-piperidine-1-carboxylic acid tert-butyl ester (8a)

4-Aminomethyl-piperidine-1-carboxylic acid *tert*-butyl ester (10.0 g. 46.7 mmol), 4-bromochlorobenzene (10.7 g, 56.0 mmol) and sodium *tert*-butoxide (6.73 g, 70.0 mmol) were dissolved in toluene (150 mL). Argon was bubbled through. 4,5-Bis(diphenylphosphino)-9,9-dimethylxanthene (Xanthphos) (810 mg; 1.40 mmol) and tris(dibenzylidineacetonedipalladium (Pd₂(dba)₃) (427 mg, 0.47 mmol) were added and the mixture was stirred at 110°C for 3 hours. Aqueous sodium hydrogen carbonate was added and the mixture was filtrated. The organic layer was washed with brine, died over sodium sulphate in combination with activated charcoal. The mixture was filtered through a pad of kieselguhr and concentrated to give the crude product as an orange sticky oil. 4-[(4-Chloro-phenylamino)-methyl]-piperidine-1-carboxylic acid *tert-*butyl ester (9.1 g, 60%) was crystallized from ethyl acetate/heptane as a white solid.

Compounds 8b-8f were prepared in parallel with compound 8a from 4-aminomethylpiperidine-1-carboxylic acid *tert*-butyl ester and the various aryl halides as shown in the table below.

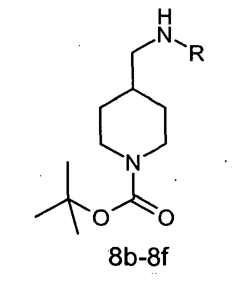

| **Compound No.** | **R** | **Aryl Halide** | **Yield (%)** |
|---|---|---|---|
| 8b | Phenyl | bromobenzene | 57 |
| 8c | 3-methoxyphenyl | 1-iodo-3-methoxybenzene | 75 |
| 8d | 3,4-methyledioxyphenyl | 4-bromo-1,2-(methylenedioxy)-benzene | 30 |
| 8e | 4-methoxyphenyl | 4-bromoanisole | 17 |
| 8f | 4-fluorophenyl | 4-bromofluorobenzene | 36 |

### Example 9

### 4-{[(4-Chloro-phenyl)-propionyl-amino]-methyl}-piperidine-1-carboxylic acid tert-butyl ester (9a)

4-[(4-Chloro-phenylamino)-methyl]-piperidine-1-carboxylic acid *tert*-butyl ester (8.8 g, 27.1 mmol) and triethylamine (4.04 mL, 28.4 mmol) were dissolved in dichloromethane (120 mL) and cooled to 0 °C. Propionyl chloride (2.53 mL, 28.4 mmol) in dichloromethane (20 mL) was added dropwise and the reaction mixture was stirred at room temperature for 2 hours. Water was added and the organic layer was washed with aqueous sodium hydrogen carbonate, water and brine. The organic layer was dried over sodium sulphate, filtered and concentrated to give 4-{[(4-chloro-phenyl)-propionyl-amino]-methyl}-piperidine-1-carboxylic acid tert-butyl ester (12 g, 100%) as a yellow oil.

Compounds 9b-9g were prepared in parallel with compound 9a as shown in the table below.

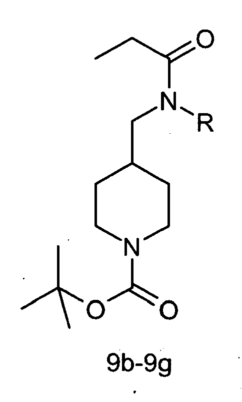

| **Compound No.** | **R** | **Starting material** | **Yield (%)** |
|---|---|---|---|
| 9b | H | 4-Aminomethylpiperidine-1-carboxylic acid tert-butyl ester | 84 |
| 9c | Phenyl | 8b | 100 |
| 9d | 3-methoxyphenyl | 8c | 100 |
| 9e | 3,4-methyledioxyphenyl | 8d | 56 |
| 9f | 4-methoxyphenyl | 8e | 82 |
| 9g | 4-fluorophenyl | 8f | 100 |

### 4-[(Acetyl-phenyl-amino)-methyl]-piperidine-1-carboxylic acid tert-butyl ester (9h)

Was prepared according to Example 9 from 8b and acetyl chloride instead of propionyl chloride.

### Example 10

### N-(4-Chloro-phenyl)-N-piperidin-4-ylmethyl-propionamide (10a)

4-{[(4-Chloro-phenyl)-propionyl-amino]-methyl}-piperidine-1-carboxylic acid *tert*-butyl ester (9a) (8.80 g, 23.1 mmol) was dissolved in dichloromethane (150 mL) and cooled to 0°C. Trifluoroacetic acid (50 mL, 673 mmol) in dichlormethane (50 mL) was added drop wise and the mixture was stirred for one hour at room temperature. The reaction mixture was concentrated and triturated with toluene, ethylacetate and diethylether. The resulting solid was filtered and washed with diethyl ether to give N-(4-chlorophenyl)-*N*-piperidin-4-ylmethyl-propionamide (9.2 g, 100%) as the trifluroacetic acid salt.

Compounds 10b-10g were prepared in parallel with compound 10a as shown in the table below.

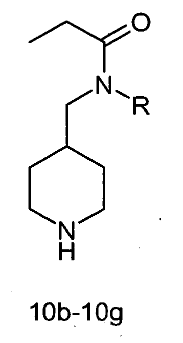

| **Compound No.** | **R** | **Starting material** | **Yield (%)** |
|---|---|---|---|
| 10b | Benzyl | 7 | 65 |
| 10c | Phenyl | 9c | 92 |
| 10d | 3-methoxyphenyl | 9d | 100 |
| 10e | 3,4-methyledioxyphenyl | 9e | 100 |
| 10f | 4-methoxyphenyl | 9f | 100 |
| 10g | 4-fluorophenyl | 9g | 75 |
| 10h | Phenyl | 9h | 88 |

### Example 11

### Methanesulfonic acid 2-(3-fluoro-phenyl)-ethyl ester (11)

3-Fluorophenethyl alcohol (0.89 mL, 7.13 mL) and pyridine (1.16 mL, 14.3 mmol) were dissolved in dichloromethane (10 mL). Methanesulfonyl chloride (0.67 mL, 8.56 mL) was added and the reaction mixture was stirred at room temperature over night. The reaction mixture was poured into ice water and extracted with dichloromethane. The combined organic phases were washed with hydrochloric acid (1 M in water) and brine, dried over sodium sulphate, filtered and concentrated. The crude product was used without further purification.

### Example 12

### N-(4-Chloro-phenyl)-N-(1-phenethyl-piperidin-4-ylmethyl)-propionamide fumaric acid salt (12a)

*N*-(4-Chloro-phenyl)-N-piperidin-4-ylmethyl-propionamide (4.0 g, 10.1 mmol) and potassium carbonate (3.64 g, 21.3 mmol) were dissolved in 2-butanone (60 mL). (2-Bromoethyl)benzene (1.46 mL, 10.6 mmol) was added and the mixture was stirred at 50°C over night. Aqueous sodium hydrogen carbonate was added and the aqueous phase was extracted with dichloromethane. The combined organic phases were dried over sodium sulphate, filtered and concentrated. The crude product was purified by flash chromatography using dichloromethane/methanol as eluent to give N-(4-chlorophenyl)-N-(1-phenethyl-piperidin-4-ylmethyl)-propionamide (3.0 g, 77%) The product was dissolved in dichloromethane and fumaric acid dissolved in methanol was added and the mixture concentrated to give the product as the fumaric acid salt as a white solid. (3.48 g, 70%)
Mp = 165-166°C LC-ESI-HRMS of [M+H]+ shows 385.2048 Da. Calc. 385.204666 Da, dev. 0.3 ppm

Compounds 12b-12t were prepared in parallel with compound 12a as shown in the table below.

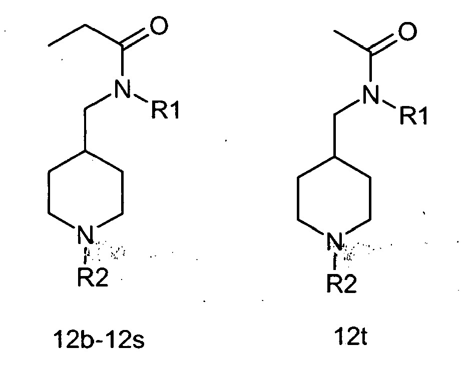

| **Cmpd. No.** | **R1** | **R2** | **Starting material 1** | **Starting material 2** | **Yield (%)** |
|---|---|---|---|---|---|
| 12b | 3-methoxyphenyl | 2-phenylethyl | 10d | (2-bromoethyl)-benzene | 5 |
| 12c | 3,4-methylene-dioxyphenyl | 2-phenylethyl | 10e | (2-bromoethyl)-benzene | 45 |
| 12d | 3,4-methylene-dioxyphenyl | 2-(4-chlorophenyl)ethyl | 10e | 4-chlorophenethyl bromide | 18 |
| 12e | 4-methoxyphenyl | 2-phenylethyl | 10f | (2-bromoethyl)-benzene | 24 |
| 12f | 4-methoxyphenyl | 2-(4-chlorophenyl)ethyl | 10f | 4-chlorophenethyl bromide | 40 |
| 12g | 3-methoxyphenyl | 2-(4-chlorophenyl)ethyl | 10d | 4-chlorophenethyl bromide | 6 |
| 12h | phenyl | 1-butene-4-yl | 10c | 4-bromo-1-butene | 51 |
| 12i | phenyl | cinnamyl | 10c | cinnamyl bromide | 30 |
| 12j | Phenyl | 2-(4-fluorophenyl)ethyl | 10c | 4-fluorophenethyl bromide | 53 |
| 12k | phenyl | 2-(3-fluorophenyl)ethyl | 10c | 11 | 26 |
| 12l | 4-fluorophenyl | 2-phenylethyl | 10g | (2-bromoethyl)-benzene | 49 |
| 12m | 4-fluorophenyl | 2-(3-fluorophenyl)ethyl | 10g | 11 | 17 |
| 12n | 4-fluorophenyl | 4-methoxyphen ethyl | 10g | 4-methoxyphenethyl bromide | 27 |
| 12o | 4-fluorophenyl | 2-(4-fluorophenyl)ethyl | 10g | 4-fluorophenethyl bromide | 52 |
| 12p | 3-fluorophenyl | 2-phenylethyl | 6 | (2-bromoethyl)-benzene | 45 |
| 12q | 3-fluorophenyl | 2-(4-fluorophenyl)ethyl | 6 | 4-fluorophenethyl bromide | 49 |
| 12r | 3-fluorophenyl | 2-(3-fluorophenyl)ethyl | 6 | 11 | 24 |
| 12s | benzyl | 2-phenylethyl | 10b | (2-bromoethyl)-benzene | 25 |
| 12t | phenyl | 2-phenylethyl | 10h | (2-bromoethyl)-benzene | 37 |

### N-(3-Methoxy-phenyl)-N-(1-phenethyl-piperidin-4-ylmethyl)-propionamide fumaric acid salt (12b)

Mp.139-141°C LC-ESI-HRMS of [M+H]+ shows 381.2538 Da. Calc. 381.254203 Da, dev. -1.1 ppm

### N-Benzo[1,3]dioxol-5-yl-N-(1-phenethyl-piperidin-4-ylmethyl)-propionamide fumaric acid salt (12c)

Mp. = 85-88°C. LC-ESI-HRMS of [M+H]+ shows 395.2325 Da. Calc. 395.233468 Da, dev. -2.4 ppm

### N-Benzo[1,3]dioxol-5-yl-N-{1-[2-(4-chloro-phenyl)-ethyl]-piperidin-4-ylmethyl}-propionamide fumaric acid salt (12d)

Mp = 125-128°C LC-ESI-HRMS of [M+H]+ shows 429.1958 Da. Calc. 429.194496 Da, dev. 3 ppm

### N-(4-Methoxy-phenyl)-N-(1-phenethyl-piperidin-4-ylmethyl)-propionamide fumaric acid salt (12e)

Mp = 114-116°C LC-ESI-HRMS of [M+H]+ shows 381.2539 Da. Calc. 381.254203 Da, dev. -0.8 ppm

### N-{1-[2-(4-Chloro-phenyl)-ethyl]-piperidin-4-ylmethyl}-N-(4-methoxy-phenyl)-propionamide fumaric acid salt (12f)

LC-ESI-HRMS of [M+H]+ shows 415.2156 Da. Calc. 415.215231 Da, dev. 0.9 ppm

### N-{1-[2-(4-Chloro-phenyl)-ethyl]-piperidin-4-ylmethyl}-N-(3-methoxy-phenyl)-propionamide fumaric acid salt (12g)

Mp. = 136-139°C LC-ESI-HRMS of [M+H]+ shows 415.2166 Da. Calc. 415.215231 Da, dev. 3.3 ppm

### N-(1-But-3-enyl-piperidin-4-ylmethyl)-N-phenyl-propionamide fumaric acid salt (12h)

Mp. = 113-116°C LC-ESI-HRMS of [M+H]+ shows 301.2268 Da. Calc. 301.227988 Da, dev. -3.9 ppm

### N-Phenyl-N-[1-((E)-3-phenyl-allyl)-piperidin-4-ylmethyl]-propionamide fumaric acid salt (12i)

Mp. = 38-41 °C LC-ESI-HRMS of [M+H]+ shows 363.2436 Da. Calc. 363.243638 Da, dev. -0.1 ppm

### N-{1-[2-(4-Fluoro-phenyl)-ethyl]-piperidin-4-ylmethyl}-N-phenyl-propionamide fumaric acid salt (12j)

Mp. = 141-143°C LC-ESI-HRMS of [M+H]+ shows 369.2356 Da. Calc. 369.234216 Da, dev. 3.7 ppm

### N-{1-[2-(3-Fluoro-phenyl)-ethyl]-piperidin-4-ylmethyl}-N-phenyl-propionamide fumaric acid salt (12k)

Mp. = 142-145°C LC-ESI-HRMS of [M+H]+ shows 369.2339 Da. Calc. 369.234216 Da, dev. -0.9 ppm

### N-(4-Fluoro-phenyl)-N-(1-phenethyl-piperidin-4-ylmethyl)-propionamide fumaric acid salt (12l)

Mp. = 126-128°C LC-ESI-HRMS of [M+H]+ shows 369.233 Da. Calc. 369.234216 Da, dev. -3.3 ppm

### N-(4-Fluoro-phenyl)-N-{1-[2-(3-fluoro-phenyl)-ethyl]-piperidin-4-ylmethyl}-propionamide fumaric acid salt (12m)

Mp. = 130-133°C LC-ESI-HRMS of [M+H]+ shows 387.2245 Da. Calc. 387.224794 Da, dev. -0.8 ppm

### N-(4-Fluoro-phenyl)-N-{1-[2-(4-methoxy-phenyl)-ethyl]-piperidin-4-ylmethyl}-propionamide fumaric acid salt (12n)

Mp. = 132-134°C LC-ESI-HRMS of [M+H]+ shows 399.2453 Da. Calc. 399.244781 Da, dev. 1.3 ppm

### N-(4-Fluoro-phenyl)-N-{1-[2-(4-fluoro-phenyl)-ethyl]-piperidin-4-ylmethyl}-propionamide fumaric acid salt (12o)

Mp. = 145-147°C LC-ESI-HRMS of [M+H]+ shows 387.225 Da. Calc. 387.224794 Da, dev. 0.5 ppm

### N-(3-Fluoro-phenyl)-N-(1-phenethyl-piperidin-4-ylmethyl)-propionamide fumaric acid salt (12p)

Mp. = 58-60 LC-ESI-HRMS of [M+H]+ shows 369.2329 Da. Calc. 369.234216 Da, dev. -3.6 ppm

### N-(3-Fluoro-phenyl)-N-{1-[2-(4-fluoro-phenyl)-ethyl]-piperidin-4-ylmethyl}-propionamide fumaric acid salt (12q)

Mp. = 65-67°C LC-ESI-HRMS of [M+H]+ shows 387.2256 Da. Calc. 387.224794 Da, dev. 2.1 ppm

### N-(3-Fluoro-phenyl)-N-{1-[2-(3-fluoro-phenyl)-ethyl]-piperidin-4-ylmethyl}-propionamide fumaric acid salt (12r)

Mp. = 142-145°C. LC-ESI-HRMS of [M+H]+ shows 387.2233 Da. Calc. 387.224794 Da, dev. -3.9 ppm

### N-Benzyl-N-(1-phenethyl-piperidin-4-ylmethyl)-propionamide fumaric acid salt (12s)

Mp.= 46-49°C LC-ESI-HRMS of [M+H]+ shows 365.2591 Da. Calc. 365.259288 Da, dev. -0.5 ppm

### N-(1-Phenethyl-piperidin-4-ylmethyl)-N-phenyl-acetamide fumaric acid salt (12t)

Mp. = 207-208°C LC-ESI-HRMS of [M+H]+ shows 337.2284 Da. Calc. 337.227988 Da, dev. 1.2 ppm

### N-(1-Phenethyl-piperidin-4-yl-methyl)-N-phenyl-propionamide hydrochloric acid salt (12u)

A mixture of *N*-phenyl-*N*-piperidin-4-yl-methyl-propionamide (0.30 g, 1.22 mmol), 2-phenethyl bromide (0.27 g, 1.46 mmol), sodium hydride, 60% in mineral oil (0.06 g, 1.5 mmol) and tetrahydrofuran (15 ml) was stirred at 15 h at 60°C followed by 70 h at room temperature. Water (20 ml) was added and the mixture was extracted with diethylether (2 x 30 ml). The diethylether phase was washed with water (20 ml). The hydrochloride was precipitated by addition of hydrogen chloride in ethanol. The gum-like solid was further purified by addition of water (20 ml) and was washed with diethylether (20 ml). The aqueous mixture was made alkaline by adding aqueous sodium hydroxide (10 ml, 1 M) and was extracted with diethylether (2 x 20 ml). The hydrochloride was was precipitated by addition of hydrogen chloride in ethanol. Yield 0.27 g (63%). Mp = 152°C.

Compounds 11v-11y were prepared in parallel with compound 11 u as shown in the table below.

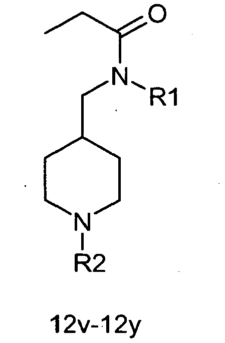

| **Compound No.** | **R1** | **R2** | **Starting material 1** | **Starting material 2** | **Yield (%)** |
|---|---|---|---|---|---|
| 12v | phenyl | benzyl | 10c | benzyl bromide | 24 |
| 12x | phenyl | 4-chlorobenzyl | 10c | 4-chlorobenzyl-bromide | 10 |
| 12y | phenyl | 2-(4-chlorophenyl)-ethyl | 10c | 4-chlorophenethyl bromide | 28 |

### N-(1-Benzyl-piperidin-4-ylmethyl)-N-phenyl-propionamide fumaric acid salt (12v)

Mp. = 58-60°C LC-ESI-HRMS of [M+H]+ shows 337.2267 Da. Calc. 337.227988 Da, dev. -3.8 ppm

### N-[1-(4-Chloro-benzyl)-piperidin-4-ylmethyl]-N-phenyl-propionamide fumaric acid salt (12x)

Mp. = 148-150°C LC-ESI-HRMS of [M+H]+ shows 371.1897 Da. Calc. 371.189016 Da, dev. 1.8 ppm

### N-{1-[2-(4-Chloro-phenyl)-ethyl]-piperidin-4-ylmethyl}-N-phenyl-propionamide fumaric acid salt (12y)

Mp. = 157-158°C LC-ESI-HRMS of [M+H]+ shows 385.2028 Da. Calc. 385.204666 Da, dev. -4.8 ppm

### Example 13

### N-Phenyl-N-piperidin-4-yl-methyl-propionamide (13)

A mixture of 4-[(phenyl-propionyl-amino)-methyl]-piperidine-1-carboxylic acid *tert*-butyl ester (4.85 g, 14 mmol) and hydrogen chloride in acetic acid (20 ml, 4 M) was stirred at room temperature for 5 h. The solvent was evaporated. Aqueous sodium hydroxide (20 ml, 1 M) was added and the mixture was extracted with ethylacetate (3 x 30 ml) followed by dichloromethane (30 ml). The crude mixture was purified by silica gel chromatography, using a solvent mixture of dichloromethane, methanol and concentrated aqueous ammonia (6:1 +1 %) as eluent. Yield 0.30 g (9%) as colourless oil.

### Example 14

### 4-[(Phenyl-propionyl-amino)-methyl]-piperidine-1-carboxylic acid tert-butyl ester (14)

A mixture of 4-phenylaminomethyl-piperidine-1-carboxylic acid *tert*-butyl ester (4.07 g, 14 mmol), propionic anhydride (2.19 g, 16.8 mmol), triethylamine (1.7 g, 16.8 mmol) and tetrahydrofuran (80 ml) was stirred at reflux for 15 h. Water (100 ml) was added and the mixture was extracted with diethylether (2 x 60 ml). The diethylether phase was washed with water (2 x 50 ml). Yield 5.05 g (100%).

### Example 15

### 4-Phenylaminomethyl-piperidine-1-carboxylic acid tert-butyl ester (15)

A mixture of 4-aminomethyl-piperidine-1-carboxylic acid *tert*-butyl ester (10.13 g, 47.3 mmol), bromobenzene (8.2 g, 52 mmol), palladacycle (0.30 g, 0.32 mmol), potassium tert-butoxide (11.1 g, 99. 3 mmol) and dioxane (50 ml) was stirred at 100 °C for 15 h Water (100 ml) was added followed by extraction with diethylether (3 x 50 ml). The diethylether phase was washed with water (2 x 50 ml). The mixture was dried and evaporated. The crude mixture was purified by silica gel chromatography, using a solvent mixture of dichloromethane, methanol and concentrated aqueous ammonia (6:1+1%) as eluent. Yield 6.18 g (45%).

### Test Example

### Binding data

The compounds have been tested in binding assays using human recombinant opiate δ-, κ- and µ receptors. The assays were conducted as previously described by Simonin F et al [Simonin F et al, Mol. Pharmacol., 46(6), 1015-21, 1994], Simonin F et al [Simonin F et al, Proc. Natl. Acad. Sci. USA, 92(15), 7006-10, 1995], and Wang JB et al [Wang JB et al, FEBS Lett., 348(1), 75-9,1994],

The test results are presented in Table 1 below.

**Table 1**

| Compound | δ | κ | µ |
|---|---|---|---|
| **12j** | 28% inhib at 10 µM | 41% inhib at 10 µM | 77% inhib at 10 µM |
| **12r** | 4% inhib at 10 µM | 68% inhib at 10 µM | 94% inhib at 10 µM |
| **12u** | 12% inhib at 10 µM | 46% inhib at 10 µM | Kᵢ = 0.0373 µM |

Furthermore, one compound, compound **12u,** was tested for functional activity in guinea pig ileum. The assay was conducted as previously described by Maguire P et al [Maguire P et al, Eur. J. Pharmacol., 213(2), 219-25, 1992].
Compound **12u** showed significant µ agonist activity with an EC₅₀ of 92 nM.

### In vitro inhibition activity

A number of compounds were tested for their ability to inhibit the reuptake of the monoamine neurotransmitters dopamine (DA) noradrenaline (NA) and serotonine (5-HT) in synaptosomes as described in WO 97/16451.

The test values are given as IC₅₀ (the concentration (µM) of the test substance which inhibits the specific binding of ³H-DA, ³H-NA, or ³H-5-HT by 50%).

Test results obtained by testing selected compounds of the present invention appear from the below table:

**Table 2**

| Test compound | DA-uptake IC₅₀(µM) | NA-uptake IC₅₀(µM) | 5-HT-uptake IC₅₀(µM) |
|---|---|---|---|
| **12j** | 0.48 | 0.28 | 5.5 |
| **12r** | 1.6 | 0.34 | 4.4 |
| **12u** | 0.73 | 0.37 | 1.3 |

## Claims

1. A compound of Formula I: 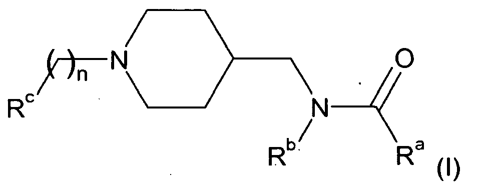 any of its isomers or any mixture of its isomers, or a pharmaceutically acceptable salt thereof;
wherein
n is 1 or 2;
R^{a} represents hydrogen, alkyl or R^{d};
which alkyl is optionally substituted with one or more substituents independently selected from the group consisting of:
halo, trifluoromethyl, trifluoromethoxy, cyano, hydroxy, amino, nitro, alkoxy, cycloalkoxy, alkyl, cycloalkyl, cycloalkylalkyl, alkenyl and alkynyl;
R^{b} represents R^{e} or -CH₂-R^{e};
R^{c} represents R^{f}, -CH=CH-R^{f} or -CH=CH₂; and
R^{d}, R^{e} and R^{f} independent of each other represent an aryl group;
which aryl group is optionally substituted with one or more substituents independently selected from the group consisting of:
halo, trifluoromethyl, trifluoromethoxy, cyano, nitro, hydroxy, alkoxy, cycloalkoxy, alkoxyalkyl, cycloalkoxyalkyl, methylenedioxy, ethylenedioxy, alkyl, cycloalkyl, cycloalkylalkyl, alkenyl, alkynyl, sulfanyl, thioalkoxy, -NR'R", -(C=O)NR'R" or -NR'(C=O)R";
wherein R' and R" independent of each other are hydrogen or alkyl.

2. The compound according to claim 1 being a compound of formula II: 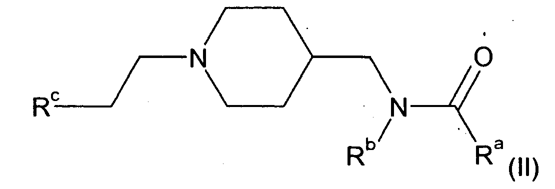 any of its isomers or any mixture of its isomers, or a pharmaceutically acceptable salt thereof;
wherein
R^{a} represents hydrogen, alkyl or R^{d};
which alkyl is optionally substituted with one or more substituents independently selected from the group consisting of:
halo, trifluoromethyl, trifluoromethoxy, cyano, hydroxy, amino, nitro, alkoxy, cycloalkoxy, alkyl, cycloalkyl, cycloalkylalkyl, alkenyl and alkynyl; and
R^{b}, R^{c} and R^{d} independent of each other represent an aryl group;
which aryl group is optionally substituted with one or more substituents independently selected from the group consisting of:
halo, trifluoromethyl, trifluoromethoxy, cyano, nitro, hydroxy, alkoxy, cycloalkoxy, alkoxyalkyl, cycloalkoxyalkyl, methylenedioxy, ethylenedioxy, alkyl, cycloalkyl, cycloalkylalkyl, alkenyl, alkynyl, sulfanyl, thioalkoxy, -NR'R", -(C=O)NR'R" or -NR'(C=O)R";
wherein R' and R" independent of each other are hydrogen or alkyl.

3. The compound according to claims 1 or 2, wherein
R^{a} represents alkyl.

4. The compound according to any one of claims 1-3, wherein
R^{b} represents an optionally substituted phenyl.

5. The compound according to any one of claims 1-4, wherein
R^{c} represents an optionally substituted phenyl.

6. A compound of claim 1 which is
*N*-(4-Chloro-phenyl)-*N*-(1-phenethyl-piperidin-4-ylmethyl)-propionamide;
*N*-(3-Methoxy-phenyl)-*N*-(1-phenethyl-piperidin-4-ylmethyl)-propionamide;
N-Benzo[1,3]dioxol-5-yl-*N*-(1-phenethyl-piperidin-4-ylmethyl)-propionamide;
N-Benzo[1,3]dioxol-5-yl-*N*-{1-[2-(4-chloro-phenyl)-ethyl]-piperidin-4-ylmethyl}-propionamide;
N-(4-Methoxy-phenyl)-*N*-(1-phenethyl-piperidin-4-ylmethyl)-propionamide;
N-{1-[2-(4-Chloro-phenyl)-ethyl]-piperidin-4-ylmethyl}-N-(4-methoxy-phenyl)-propionamide;
N-{1-[2-(4-Chloro-phenyl)-ethyl]-piperidin-4-ylmethyl}-N-(3-methoxy-phenyl)-propionamide ;
N-(1-But-3-enyl-piperidin-4-ylmethyl)-N-phenyl-propionamide;
N-Phenyl-N-[1-((E)-3-phenyl-allyl)-piperidin-4-ylmethyl]-propionamide;
N-{1-[2-(4-Fluoro-phenyl)-ethyl]-piperidin-4-ylmethyl}-N-phenyl-propionamide;
N-{1-[2-(3-Fluoro-phenyl)-ethyl]-piperidin-4-ylmethyl}-N-phenyl-propionamide;
N-(4-Fluoro-phenyl)-N-(1-phenethyl-piperidin-4-ylmethyl)-propionamide;
N-(4-Fluoro-phenyl)-N-{1-[2-(3-fluoro-phenyl)-ethyl]-piperidin-4-ylmethyl}-propionamide;
N-(4-Fluoro-phenyl)-N-{1-[2-(4-methoxy-phenyl)-ethyl]-piperidin-4-ylmethyl}-propionamide;
N-(4-Fluoro-phenyl)-N-(1-[2-(4-fluoro-phenyl)-ethyl]-piperidin-4-ylmethyl)-propionamide;
N-(3-Fluoro-phenyl)-N-(1-phenethyl-piperidin-4-ylmethyl)-propionamide;
N-(3-Fluoro-phenyl)-N-{1-[2-(4-fluoro-phenyl)-ethyl]-piperidin-4-ylmethyl}-propionamide;
N-(3-Fluoro-phenyl)-N-{1-[2-(3-fluoro-phenyl)-ethyl]-piperidin-4-ylmethyl}-propionamide;
N-Benzyl-N-(1-phenethyl-piperidin-4-ylmethyl)-propionamide;
N-(1-Phenethyl-piperidin-4-ylmethyl)-N-phenyl-acetamide;
*N*-(1-Phenethyl-piperidin-4-yl-methyl)-*N*-phenyl-propionamide;
N-(1-Benzyl-piperidin-4-ylmethyl)-N-phenyl-propionamide;
N-[1-(4-Chloro-benzyl)-piperidin-4-ylmethyl]-N-phenyl-propionamide;
N-{1-[2-(4-Chloro-phenyl)-ethyl]-piperidin-4-ylmethyl}-N-phenyl-propionamide;
or a pharmaceutically acceptable salt thereof.

7. A pharmaceutical composition, comprising a therapeutically effective amount of a compound of any one of claims 1-6, any of its isomers or any mixture of its isomers, or a pharmaceutically acceptable salt thereof, together with at least one pharmaceutically acceptable carrier, excipient or diluent.

8. The use of a compound according to any one of claims 1-6, any of its isomers or any mixture of its isomers, or a pharmaceutically acceptable salt thereof, for the manufacture of a pharmaceutical composition for the treatment, prevention or alleviation of a disease or a disorder or a condition of a mammal, including a human, which disease, disorder or condition is responsive to modulation of the opioid receptor.

9. The use according to claim 8, wherein the disease, disorder or condition responsive to modulation of the opioid receptor is pain, postoperative pain, chronic pain, cancer pain, neuropathic pain, pain during labour and delivery, migraine, drug addiction, heroin addiction, cocaine addiction, alcoholism, irritable bowel syndrome, constipation, nausea, vomiting, pruritic dermatoses, allergic dermatitis, atopy, eating disorders, opiate overdoses, depression, smoking, sexual dysfunction, shock, stroke, spinal damage, head trauma, diarrhoea, urinary incontinence and inflammatory reactions.

10. The compound according to any one of claims 1-6, any of its isomers or any mixture of its isomers, or a pharmaceutically acceptable salt thereof, for use as a pharmaceutical composition.

11. The compound according to any one of claims 1-6, any of its isomers or any mixture of its isomers, or a pharmaceutically acceptable salt thereof, for use in the treatment, prevention or alleviation of a disease or a disorder or a condition of a mammal, including a human, which disease, disorder or condition is responsive to modulation of the opioid receptor.

## Patentansprüche

1. Verbindung der Formel I: 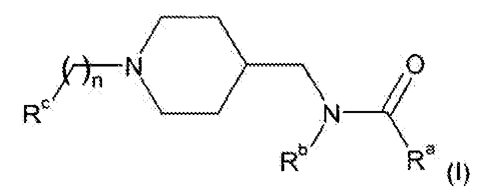 beliebige von ihren Isomeren oder eine beliebige Mischung von ihren Isomeren, oder ein pharmazeutisch verträgliches Salz davon; wobei
n 1 oder 2 ist;
R^{a} Wasserstoff, Alkyl oder R^{d} bedeutet;
wobei dieses Alkyl gegebenenfalls mit einem oder mehreren Substituenten substituiert ist, die unabhängig voneinander ausgewählt sind aus der Gruppe bestehend aus:
Halogen, Trifluormethyl, Trifluormethoxy, Cyano, Hydroxy, Amino, Nitro, Alkoxy, Cycloalkoxy, Alkyl, Cycloalkyl, Cycloalkylalkyl, Alkenyl und Alkinyl;
R^{b} R^{e} oder -CH₂-R^{e} bedeutet;
R^{c} R^{f}, -CH=CH-R^{f} oder -CH=CH₂ bedeutet; und
R^{d}, R^{e} und R^{f} unabhängig voneinander eine Arylgruppe bedeuten;
wobei diese Arylgruppe gegebenenfalls mit einem oder mehreren Substituenten substituiert ist, die unabhängig voneinander ausgewählt sind aus der Gruppe bestehend aus:
Halogen, Trifluormethyl, Trifluormethoxy, Cyano, Nitro, Hydroxy, Alkoxy, Cycloalkoxy, Alkoxyalkyl, Cycloalkoxyalkyl, Methylendioxy, Ethylendioxy, Alkyl, Cycloalkyl, Cycloalkylalkyl, Alkenyl, Alkinyl, Sulfanyl, Thioalkoxy, -NR'R", -(C=O)NR'R" oder -NR'(C=O)R";
wobei R' und R" unabhängig voneinander Wasserstoff oder Alkyl sind.

2. Verbindung nach Anspruch 1, wobei es sich um eine Verbindung der Formel II handelt: 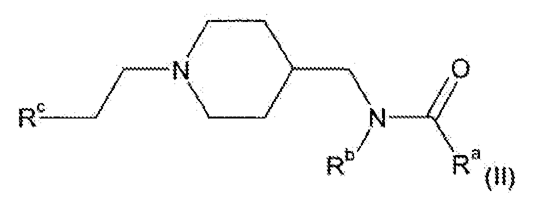 beliebige von ihren Isomeren oder eine beliebige Mischung von ihren Isomeren, oder ein pharmazeutisch verträgliches Salz davon;
wobei
R^{a} Wasserstoff, Alkyl oder R^{d} bedeutet;
wobei dieses Alkyl gegebenenfalls mit einem oder mehreren Substituenten substituiert ist, die unabhängig voneinander ausgewählt sind aus der Gruppe bestehend aus:
Halogen, Trifluormethyl, Trifluormethoxy, Cyano, Hydroxy, Amino, Nitro, Alkoxy, Cycloalkoxy, Alkyl, Cycloalkyl, Cycloalkylalkyl, Alkenyl und Alkinyl; und
R^{b}, R^{c} und R^{d} unabhängig voneinander eine Arylgruppe bedeuten;
wobei diese Arylgruppe gegebenenfalls mit einem oder mehreren Substituenten substituiert ist, die unabhängig voneinander ausgewählt sind aus der Gruppe bestehend aus:
Halogen, Trifluormethyl, Trifluormethoxy, Cyano, Nitro, Hydroxy, Alkoxy, Cycloalkyl, Alkoxyalkyl, Cycloalkoxyalkyl, Methylendioxy, Ethylendioxy, Alkyl, Cycloalkyl, Cycloalkylalkyl, Alkenyl, Alkinyl, Sulfanyl, Thioalkoxy, -NR'R", -(C=O)NR'R" oder -NR'(C=O)R";
wobei R' und R" unabhängig voneinander Wasserstoff oder Alkyl sind.

3. Verbindung nach den Ansprüchen 1 oder 2, wobei R^{a} Alkyl bedeutet.

4. Verbindung nach einem der Ansprüche 1-3, wobei R^{b} ein gegebenenfalls substituiertes Phenyl bedeutet.

5. Verbindung nach einem der Ansprüche 1-4, wobei R^{c} ein gegebenenfalls substituiertes Phenyl bedeutet.

6. Verbindung nach Anspruch 1, welche
*N*-(4-Chlor-phenyl)-*N*-(1-phenethyl-piperidin-4-ylmethyl)-propionamid;
*N*-(3-Methoxy-phenyl)-*N*-(1-phenethyl-piperidin-4-ylmethyl)-propionamid;
N-Benzo[1,3]dioxol-5-yl-*N*-(1-phenethyl-piperidin-4-ylmethyl)-propionamid;
N-Benzo[1,3]dioxol-5-yl-*N*-{1-[2-(4-chlor-phenyl)-ethyl]-piperidin-4-ylmethyl}-propionamid;
N-(4-Methoxy-phenyl)-*N*-(1-phenethyl-piperidin-4-ylmethyl)-propionamid;
N-{1-[2-(4-Chlor-phenyl)-ethyl]-piperidin-4-ylmethyl}-N-(4-methoxy-phenyl)-propionamid;
N-{1-[2-(4-Chlor-phenyl)-ethyl]-piperidin-4-ylmethyl}-N-(3-methoxy-phenyl)-propionamid;
N-(1-But-3-enyl-piperidin-4-ylmethyl)-N-phenyl-propionamid;
N-Phenyl-N-[1-((E)-3-phenyl-allyl)-piperidin-4-ylmethyl]-propionamid;
N-{1-[2-(4-Fluor-phenyl)-ethyl]-piperidin-4-ylmethyl}-N-phenyl-propionamid;
N-{1-[2-(3-Fluor-phenyl)-ethyl]-piperidin-4-ylmethyl}-N-phenyl-propionamid;
N-(4-Fluor-phenyl)-N-(1-phenethyl-piperidin-4-ylmethyl)-propionamid;
N-(4-Fluor-phenyl)-N-{1-[2-(3-fluor-phenyl)-ethyl]-piperidin-4-ylmethyl}-propionamid;
N-(4-Fluor-phenyl)-N-{1-[2-(4-methoxy-phenyl)-ethyl]-piperidin-4-ylmethyl}-propionamid;
N-(4-Fluor-phenyl)-N-{1-[2-(4-fluor-phenyl)-ethyl]-piperidin-4-ylmethyl}-propionamid;
N-(3-Fluor-phenyl)-N-(1-phenethyl-piperidin-4-ylmethyl)-propionamid;
N-(3-Fluor-phenyl)-N-{1-[2-(4-fluor-phenyl)-ethyl]-piperidin-4-ylmethyl}-propionamid;
N-(3-Fluor-phenyl)-N-{1-[2-(3-fluor-phenyl)-ethyl]-piperidin-4-ylmethyl}-propionamid;
N-Benzyl-N-(1-phenethyl-piperidin-4-ylmethyl)-propionamid;
N-(1-Phenethyl-piperidin-4-ylmethyl)-N-phenyl-acetamid;
*N*-(1-Phenethyl-piperidin-4-yl-methyl)-*N*-phenyl-propionamid;
N-(1-Benzyl-piperidin-4-ylmethyl)-N-phenyl-propionamid;
N-[1-(4-Chlor-benzyl)-piperidin-4-ylmethyl]-N-phenyl-propionamid;
N-{1-[2-(4-Chlor-phenyl)-ethyl]-piperidin-4-ylmethyl}-N-phenyl-propionamid ist;
oder ein pharmazeutisch verträgliches Salz davon.

7. Pharmazeutische Zusammensetzung, umfassend eine therapeutisch wirksame Menge von einer Verbindung nach einem der Ansprüche 1-6, beliebigen von ihren Isomeren oder einer beliebigen Mischung von ihren Isomeren, oder einem pharmazeutisch verträglichen Salz davon, zusammen mit wenigstens einem pharmazeutisch verträglichen Träger, Exzipienten oder Verdünnungsmittel.

8. Verwendung von einer Verbindung nach einem der Ansprüche 1-6, beliebigen von ihren Isomeren oder einer beliebigen Mischung von ihren Isomeren, oder einem pharmazeutisch verträglichen Salz davon, für die Herstellung einer pharmazeutischen Zusammensetzung für die Behandlung, Verhütung oder Linderung einer Krankheit oder einer Störung oder eines Zustands eines Säugers, einschließlich eines Menschen,
wobei diese Krankheit, diese Störung oder dieser Zustand auf die Modulation des Opioidrezeptors anspricht.

9. Verwendung nach Anspruch 8, wobei es sich bei der Krankheit, der Störung oder dem Zustand, die bzw. der auf die Modulation des Opioidrezeptors anspricht, um Schmerzen, postoperative Schmerzen, chronische Schmerzen, Krebsschmerzen, neuropathische Schmerzen, Schmerzen während der Wehen und der Geburt, Migräne, Drogenabhängigkeit, Heroinabhängigkeit, Kokainabhängigkeit, Alkoholismus, Reizkolon, Verstopfung, Übelkeit, Erbrechen, pruritische Dermatosen, allergische Dermatitis, Atopie, Essstörungen, Opiatüberdosen, eine Depression, Rauchen, sexuelle Dysfunktion, einen Schock, Schlaganfall, Rückmarks- bzw. Wirbelsäulenschaden, eine Kopfverletzung, Diarrhö, Harninkontinenz und Entzündungsreaktionen handelt.

10. Verbindung nach einem der Ansprüche 1-6, beliebige von ihren Isomeren oder eine beliebige Mischung von ihren Isomeren, oder ein pharmazeutisch verträgliches Salz davon, zur Verwendung als eine pharmazeutische Zusammensetzung.

11. Verbindung nach einem der Ansprüche 1-6, beliebige von ihren Isomeren oder eine beliebige Mischung von ihren Isomeren, oder ein pharmazeutisch verträgliches Salz davon, zur Verwendung bei der Behandlung, Verhütung oder Linderung einer Krankheit oder einer Störung oder eines Zustands eines Säugers, einschließlich eines Menschen, wobei diese Krankheit, diese Störung oder dieser Zustand auf die Modulation des Opioidrezeptors anspricht.

## Revendications

1. Composé de formule I : 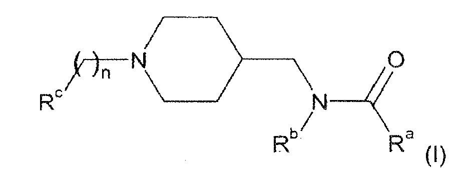 l'un quelconque de ses isomères ou tout mélange de ses isomères, ou sel pharmaceutiquement acceptable de celui-ci ;
dans laquelle
n est 1 ou 2;
R^{a} représente un hydrogène, un alkyle ou R^{d} ;
lequel alkyle est facultativement substitué par un ou plusieurs substituants indépendamment choisis dans le groupe constitué par : halogéno, trifluorométhyle, trifluorométhoxy, cyano, hydroxy, amino, nitro, alcoxy, cycloalcoxy, alkyle, cycloalkyle, cycloalkylalkyle, alcényle et alcynyle ;
R^{b} représente R^{e} ou -CH₂-R^{e} ;
R^{c} représente R^{f}, -CH=CH-R^{f} ou -CH=CH₂ ; et
R^{d}, R^{e} et R^{f}, indépendamment les uns des autres, représentent un groupe aryle ;
lequel groupe aryle est facultativement substitué par un ou plusieurs substituants indépendamment choisis dans le groupe constitué par : halogéno, trifluorométhyle, trifluorométhoxy, cyano, nitro, hydroxy, alcoxy, cycloalcoxy, alcoxyalkyle, cycloalcoxyalkyle, méthylènedioxy, éthylène-dioxy, alkyle, cycloalkyle, cycloalkylalkyle, alcényle, alcynyle, sulfanyle, thioalcoxy, -NR'R", -(C=O)NR'R" ou -NR'(C=O)R" ;
où R' et R", indépendamment l'un de l'autre, sont un hydrogène ou un alkyle.

2. Composé selon la revendication 1, qui est un composé de formule II : 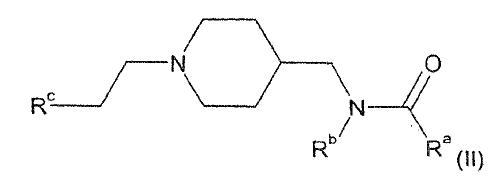 l'un quelconque de ses isomères ou tout mélange de ses isomères, ou un sel pharmaceutiquement acceptable de celui-ci ;
dans laquelle
R^{a} représente un hydrogène, un alkyle ou R^{d} ;
lequel alkyle est facultativement substitué par un ou plusieurs substituants indépendamment choisis dans le groupe constitué par : halogéno, trifluorométhyle, trifluorométhoxy, cyano, hydroxy, amino, nitro, alcoxy, cycloalcoxy, alkyle, cycloalkyle, cycloalkylalkyle, alcényle et alcynyle ; et
R^{b}, R^{c} et R^{d}, indépendamment les uns des autres, représentent un groupe aryle ;
lequel groupe aryle est facultativement substitué par un ou plusieurs substituants indépendamment choisis dans le groupe constitué par : halogéno, trifluorométhyle, trifluorométhoxy, cyano, nitro, hydroxy, alcoxy, cycloalcoxy, alcoxyalkyle, cycloalcoxyalkyle, méthylènedioxy, éthylène-dioxy, alkyle, cycloalkyle, cycloalkylalkyle, alcényle, alcynyle, sulfanyle, thioalcoxy, -NR'R", -(C=O)NR'R" ou -NR'(C=O)R" ;
où R' et R", indépendamment l'un de l'autre, sont un hydrogène ou un alkyle.

3. Composé selon les revendications 1 ou 2, dans lequel R^{a} représente un alkyle.

4. Composé selon l'une quelconque des revendications 1 à 3, dans lequel R^{b} représente un phényle facultativement substitué.

5. Composé selon l'une quelconque des revendications 1 à 4, dans lequel R^{c} représente un phényle facultativement substitué.

6. Composé selon la revendication 1, qui est le
*N*-(4-Chloro-phényl)-*N*-(1-phénéthyl-pipéridin-4-ylméthyl)-propionamide ;
*N*-(3-Méthoxy-phényl)-*N*-(1-phénéthyl-pipéridin-4-ylméthyl)-propionamide ;
*N*-Benzo[1,3]dioxol-5-yl-*N*-(1-phénéthyl-pipéridin-4-ylméthyl)-propionamide ;
*N*-Benzo[1,3]dioxol-5-yl-*N*-{1-[2-(4-chlorophényl)-éthyl]-pipéridin-4-ylméthyl}-propionamide ;
*N*-(4-Méthoxy-phényl)-*N*-(1-phénéthyl-pipéridin-4-ylméthyl)-propionamide ;
*N*-{1-[2-(4-Chloro-phényl)-éthyl]-pipéridin-4-ylméthyl}-*N*-(4-méthoxyphényl)-propionamide ;
*N*-{1-[2-(4-Chloro-phényl)-éthyl]-pipéridin-4-ylméthyl}-*N*-(3-méthoxyphényl)-propionamide ;
*N*-(1-But-3-ényl-pipéridin-4-ylméthyl)-*N*-phényl-propionamide ;
*N*-Phényl-*N*-[1-((E)-3-phényl-allyl)-pipéridin-4-ylméthyl]-propionamide ;
*N*-{1-[2-(4-Fluoro-phényl)-éthyl]pipéridin-4-ylméthyl}-*N*-phénylpropionamide ;
*N*-{1-[2-(3-Fluoro-phényl)-éthyl]-pipéridin-4-ylméthyl}-*N*-phénylpropionamide ;
*N*-(4-Fluoro-phényl)-*N*-(1-phénéthyl-pipéridin-4-ylméthyl)-propionamide ;
*N*-(4-Fluoro-phényl)-*N*-{1-[2-(3-fluoro-phényl)-éthyl]-pipéridin-4-ylméthyl}-propionamide ;
*N*-(4-Fluoro-phényl)-*N*-{1-[2-(4-méthoxy-phényl)-éthyl]-pipéridin-4-ylméthyl]-propionamide ;
*N*-(4-Fluoro-phényl)-*N*-{1-[2-(4-fluoro-phényl)-éthyl]-pipéridin-4-ylméthyl}-propionamide ;
*N*-(3-Fluoro-phényl)-*N*-(1-phénéthyl-pipéridin-4-ylméthyl)-propionamide ;
*N*-(3-Fluoro-phényl)-*N*-{1-[2-(4-fluoro-phényl)-éthyl]-pipéridin-4-ylméthyl}-propionamide ;
*N*-(3-Fluoro-phényl)-*N*-{1 -[2-(3-fluoro-phényl)-éthyl]-pipéridin-4-ylméthyl}-propionamide ;
*N*-Benzyl-*N*-(1-phénéthyl-pipéridin-4-ylméthyl)-propionamide ;
*N*-(1-Phénétyl-pipéridin-4-ylméthyl)-*N*-phényl-acétamide ;
*N*-(1-Phénéthyl-pipéridin-4-yl-méthyl)-*N*-phényl-propionamide ;
*N*-(1-Benzyl-pipéridin-4-ylméthyl)-*N*-phényl-propionamide ;
*N*-[1-(4-Chloro-benzyl)-pipéridin-4-ylméthyl]-*N*-phényl-propionamide ;
*N*-{1-[2-(4-Chloro-phényl)-éthyl]-pipéridin-4-ylméthyl}-*N*-phényl-propionamide ;
ou un sel pharmaceutiquement acceptable de celui-ci.

7. Composition pharmaceutique, comprenant une quantité thérapeutiquement efficace d'un composé selon l'une quelconque des revendications 1 à 6, de l'un quelconque de ses isomères ou de tout mélange de ses isomères, ou d'un sel pharmaceutiquement acceptable de celui-ci, conjointement avec au moins un véhicule, excipient ou diluant pharmaceutiquement acceptable.

8. Utilisation d'un composé selon l'une quelconque des revendications 1 à 6, de l'un quelconque de ses isomères ou de tout mélange de ses isomères, ou d'un sel pharmaceutiquement acceptable de celui-ci, pour la fabrication d'une composition pharmaceutique pour le traitement, la prévention ou l'atténuation d'une maladie ou d'un trouble ou d'une affection d'un mammifère, incluant un être humain, laquelle maladie, lequel trouble ou laquelle affection répond à une modulation du récepteur opioïde.

9. Utilisation selon la revendication 8, dans laquelle la maladie, le trouble ou l'affection répondant à une modulation du récepteur opioïde est une douleur, une douleur postopératoire, une douleur chronique, une douleur cancéreuse, une douleur neuropathique, une douleur pendant le travail et la délivrance, une migraine, une toxicomanie, une héroïnomanie, une cocaïnomanie, un alcoolisme, un syndrome du côlon irritable, une constipation, une nausée, un vomissement, des dermatoses prurigineuses, une dermatite allergique, une atopie, des troubles de l'alimentation, des overdoses aux opiacés, une dépression, un tabagisme, un dysfonctionnement sexuel, un état de choc, un accident vasculaire cérébral, une lésion médullaire, un traumatisme crânien, une diarrhée, une incontinence urinaire et des réactions inflammatoires.

10. Composé selon l'une quelconque des revendications 1 à 6, l'un quelconque de ses isomères ou tout mélange de ses isomères, ou sel pharmaceutiquement acceptable de celui-ci, pour une utilisation sous la forme d'une composition pharmaceutique.

11. Composé selon l'une quelconque des revendications 1 à 6, l'un quelconque de ses isomères ou tout mélange de ses isomères, ou sel pharmaceutiquement acceptable de celui-ci, pour une utilisation dans le traitement, la prévention ou l'atténuation d'une maladie ou d'un trouble ou d'une affection d'un mammifère, incluant un être humain, laquelle maladie, lequel trouble ou laquelle affection répond à une modulation du récepteur opioïde.
